# EUROPEAN PATENT APPLICATION

(11) **EP 0 565 996 A1**
(43) Date of publication of application: **20.10.1993**
(21) Application number: 93105683.2
(22) Date of filing: 06.04.1993
(51) Int. Cl.: A61M 25/01

(54) **Vascular dilatation method and apparatus for facilitating dilatition balloon exchange**

(30) Priority: 07.04.1992 US 864507; 09.09.1992 US 942598
(71) Applicant: Cordis Corporation, Miami Lakes Florida 33014 (US)
(72) Inventor: Johnson, Kirk, Miami Lakes, FL 33014 (US)
(74) Representative: KUHNEN, WACKER & PARTNER

(57) **Abstract**

An embodiment for facilitating dilatution balloon exchange employs a holding balloon (58) within a guiding catheter (50) having a generally annular or toroidal configuration, defining a central opening through which a balloon guidewire (52) can be threaded, the balloon (58) operating to constrict down upon and circumferentially grip the balloon guidewire (52) when the balloon (58) is inflated with the balloon guidewire (52) extending through its central opening.

## Description

### Cross Reference to Related Application

This a continuation-in-part based on United States patent application Serial No. 07/864,507, filed April 7, 1992, by Kirk Johnson, and assigned to the assignee of the present application.

### Background of the Invention

### 1. Technical Field

This invention relates generally to the field of cardiac catheterization, and more specifically to new apparatus and method for facilitating balloon exchange during dilatation procedures such as angioplasty.

### 2. Background Art

Cardiac catheterization procedures are well known for diagnosis and therapy of lesions in the cardiovascular system. One such procedure is angioplasty, for eliminating or ameliorating the vascular plaque blockage or constriction in blood vessels associated with the provision of the heart's blood supply. In an angioplasty procedure, an expandable balloon is introduced into the patient's arterial system and advanced until it is positioned in the region of the blockage or constriction. Once so positioned, the balloon is expanded by filling it with a liquid. In successful procedures, the expandable balloon presses outwardly against the walls of the artery and expands the artery to a degree to which the artery is either partially or totally re-opened to blood flow.

A typical angioplasty procedure, and components used in practicing the procedure, are now described.

Prior to initiating the angioplasty procedure, a guiding catheter is placed typically via the femoral artery into the aorta and its tip is engaged into the coronary arteries which branch from the aorta. This entrance into the coronary artery is called the osteum. Once placed, the guiding catheter acts as a conduit to access the coronary arteries with a balloon guidewire and balloon catheter. The guiding catheter is a portion of plastic tubing having a length of about 95 centimeters, an inside diameter of about .08 inches, and an outside diameter of about 2.5 millimeters.

The physician threads a balloon catheter onto a balloon guidewire. This operation takes place external to the patient.

The balloon guidewire is a piece of stainless steel and platinum wire, approximately 175 centimeters in length, and about 0.010-0.018 inches in diameter. The soft distal tip of the guidewire can be shaped to form a "J" configuration. This "J" shape allows the physician to steer the wire by twisting the proximal extremity of the wire while advancing or retracting the wire.

The balloon catheter is an elongated flexible plastic member defining two longitudinal passages and having a balloon located near its distal end. One longitudinal opening defines a sleeve through which the balloon guidewire can be passed. The other longitudinal passage defines a conduit communicating with the interior of the balloon and through which inflation fluid can be injected to inflate the balloon.

Among the types of balloon catheters is one of a type in which the two longitudinal passages are generally side by side and parallel. In another type of balloon catheter, the two longitudinal passages are co-axial. In this latter type, the balloon guidewire is passed down the inner passage and the inflation fluid is injected into the balloon via the outer passage.

Balloon catheters, as well as associated apparatus and method for use in angioplasty, are described in U.S. Patent 5,040,548, issued on August 20, 1991, to Yock, and U.S. Patent 4,762,129, issued on August 8, 1988. Each of these issued U.S. patents is hereby expressly incorporated by reference.

The physician passes the balloon guidewire through the appropriate one of the longitudinal passages in the balloon catheter, leaving a portion of the balloon guidewire extending from the distal end of the balloon catheter and also a portion extending from its proximal end.

This assembly is then inserted into the proximal end of the guiding catheter, distal end first. The assembly is inserted until the balloon which is attached near the distal end of the balloon catheter is near the distal end of the guiding catheter. At this point, the physician, while maintaining the balloon catheter stationary, pushes on the balloon guidewire to advance it outwardly from the distal end of the guiding catheter.

The balloon guidewire can be steered by appropriate twisting movement by the physician.

The physician steers the balloon guidewire into the chosen one of the coronary arteries, and advances it until it reaches a location of constriction which the physician desires to re-open. Carefully, the physician eases the balloon guidewire through the region of restriction until a portion of the balloon guidewire is on the opposite side of the constriction, relative to the guiding catheter.

With the balloon guidewire held stationary, the physician then advances the balloon catheter. The distal end of the balloon catheter, as it is advanced, will, of course, follow the balloon guidewire which is already in place.

The physician continues to advance the balloon until it is located in the region of constriction of the artery. With the balloon and its associated catheter held stationary, inflation fluid is injected into the conduit which communicates with the balloon, causing it to inflate. Inflation of the balloon expands the walls of the artery in the region of constriction and, in successful procedures, re-opens the artery to sufficient blood flow.

Arteries vary in size, and therefore balloon catheters having balloons of different sizes are provided for the physician's selection. These balloons, when inflated, range from about 1.5 millimeters to about 4 millimeters in diameter.

Sometimes, it is necessary for the physician to use more than one balloon to open an artery. Sometimes, the chosen balloon is too large to be advanced into the constricted area. In other instances, the first chosen balloon size, even when inflated, is not large enough to open the constricted area to the degree desired. In such cases, it is necessary to exchange one balloon for another during the same angioplasty procedure.

In order to accomplish this exchange, the balloon guidewire is left in place, and the balloon catheter is withdrawn entirely from the guiding catheter until it is completely disengaged from the balloon guidewire. A new balloon catheter, having a different sized balloon, is then re-inserted over the balloon guidewire and advanced back to the location of the constricted area, where it is used to effect the desired result.

Once the balloon guidewire is initially in place, extending past the constricted area, it is highly desirable to leave the balloon guidewire in place for the entirety of the duration of the angioplasty procedure. This means that the balloon guidewire must remain in place even during exchanges of balloons. The reason for this is that, when a foreign object, such as the balloon guidewire, is introduced into an artery, the artery walls sometimes go into spasm, and constrict generally along a substantial portion of its length. If the artery tends to contract in this way, removal of the balloon guidewire while the artery is so contracted will sometimes render it virtually impossible to re-insert the guidewire through the contracted artery.

Withdrawal of the balloon catheter, while preventing movement of the balloon guidewire, is a difficult and cumbersome procedure, requiring both a second individual, in addition to the physician, and the attachment of a removable extension to the proximal end of the guidewire.

Attachment of the extension to the guidewire during withdrawal of the balloon catheter is necessary because, if the balloon catheter were withdrawn over the proximal end of the guidewire, there would be no way that the guidewire could be manually held stationary. Attachment of the extension to the proximal end of the guidewire extends the guidewire to a point at which the proximal end of the extension still extends outwardly from the proximal end of the balloon catheter even when the balloon catheter is entirely withdrawn from the patient.

Even with the extension, the physician must enlist the aid of an assistant to manually hold stationary the end of the guidewire, preventing guidewire movement, while the physician withdraws the balloon catheter. This is a cumbersome and awkward procedure at best.

It is a general object of the present invention to provide apparatus and method for facilitating introduction and exchange of balloons in angioplasty procedures without the need for manually holding the balloon guidewire in place.

### Description of the Invention

The disadvantages of the prior art is reduced or eliminated by a novel apparatus for practicing angioplasty procedures. The apparatus includes a flexible tubular guiding catheter sized for insertion into the vascular system of a patient. A balloon guidewire is provided which is adapted for longitudinal positioning within the guiding catheter, and is long enough to extend simultaneously from both ends of the guiding catheter. A balloon catheter including a first balloon coupled near its distal end is also included. The balloon catheter defines a hollow central longitudinal passage through which the balloon guidewire can pass. The balloon catheter also defines conduit means for facilitating the transmission of inflation fluid to the balloon.

A holding balloon is disposed within the guiding catheter at a position such that, when the holding balloon is inflated, the holding balloon pinches the balloon guidewire against the interior side wall of the guiding catheter when the guidewire is located in the region of the holding balloon. Finally, the apparatus includes means for separately and independently inflating the first balloon, which is coupled to the balloon catheter and the holding balloon.

When the holding balloon is deflated, the balloon catheter and the balloon it carries can be withdrawn until the balloon catheter and the balloon are both proximal to the holding balloon, all without losing the proximal end of the balloon guidewire.

The use of such an apparatus facilitates the withdrawal of the balloon catheter from the guiding catheter without the need for manually holding the balloon guidewire stationary. This advantage in turn enables the physician to remove the balloon catheter from the balloon guidewire without the aid of an assistant and without the necessity for anyone holding the balloon guidewire. Thus, the fact that, during withdrawal of the balloon catheter, the balloon catheter covers the proximal end of the balloon guidewire is of no relevance.

This advantage is enabled by the holding balloon. The holding balloon is located near the distal end of the guiding catheter. When the physician desires to withdraw a balloon catheter, for example when a balloon exchange is needed, he withdraws the balloon catheter only so far as to place the balloon which is coupled to the balloon catheter proximal to the holding balloon, and no further. He then inflates the holding balloon, which pinches the balloon guidewire against an interior wall surface of the guiding catheter. The balloon guidewire is thus held stationary by friction, and need not be manually held to maintain its stationary position. In this configuration, the physician can then simply remove the balloon catheter by pulling it off the balloon guidewire, and need not be concerned about the fact that he cannot, during much of the withdrawing operation, directly grasp the balloon guidewire.

According to a more specific aspect of the invention, the holding balloon is affixed to an interior surface of the guiding catheter near its distal end. A conduit is provided communicating with the interior of the holding balloon, and extending to a point external to the guiding catheter, at which point inflation fluid can be inserted into the conduit to expand the balloon when desired.

In accordance with a more specific aspect, the conduit is integrally formed in the wall of the guiding catheter.

In another embodiment, the holding balloon is generally annular in shape and is anchored circumferentially about the inside of the guiding catheter. A hole is defined in the center of the holding balloon and the balloon guidewire extends through it. When it is desired to restrict longitudinal movement of the balloon guidewire, the annular holding balloon is inflated with fluid, which causes it to press circumferentially about the outer surface of the balloon guidewire.

This invention will be understood in more detail by reference to the following detailed description, and to the drawings in which:

### Brief Description of the Drawings

Figure 1 is an elevational view, partly in cross-section, illustrating the present invention;
Figure 1A is a detail elevational view, partly in cross-section, showing the portion of the apparatus of Figure 1 indicated by the inset 1A;
Figure 2 is an elevational view, partly in cross-section, showing the apparatus of Figure 1 in a different operative configuration;
Figure 3 is an elevational view, partly in cross-section, illustrating an alternate embodiment of the present invention.

### Best Mode for Carrying Out the Invention

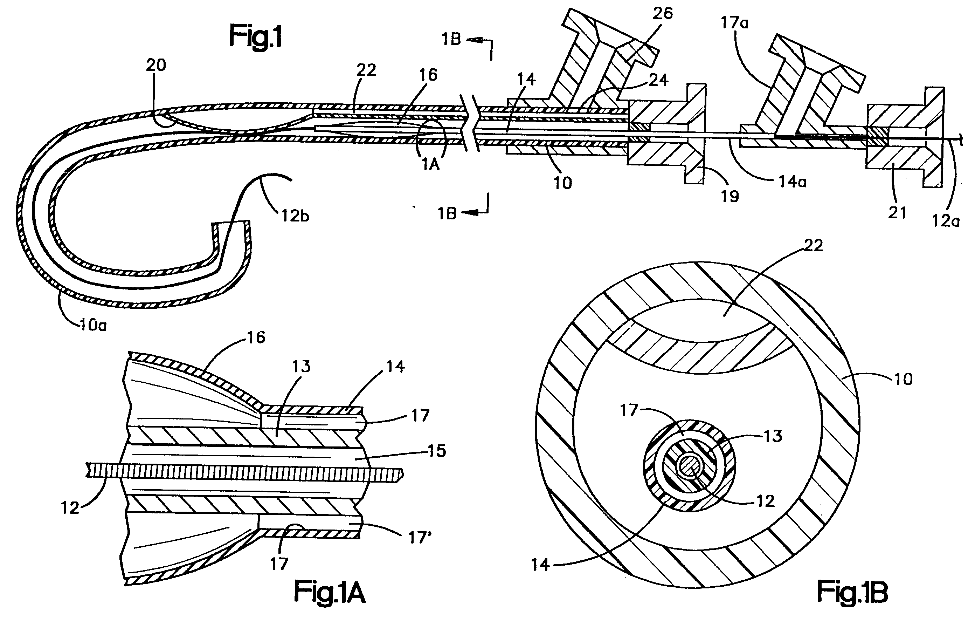

Figure 1 shows the relevant portions of apparatus suitable for practicing angioplasty, which apparatus incorporates the present invention.

Figure 1 shows a guiding catheter 10. The guiding catheter 10 is to be presumed as already positioned within the patient's arterial system as described above.

A balloon guidewire 12 is also illustrated. The balloon guidewire extends outwardly from the proximal end of the guiding catheter 10, near the right-hand portion of Figure 1, at reference character 12a. The balloon guidewire 12 also extends entirely through the guiding catheter and extends outwardly from the distal end 10a of the guiding catheter with a portion indicated at reference character 12b.

A balloon catheter 14 is illustrated with the balloon guidewire 12 passing through a longitudinal passage extending through the balloon catheter. Preferably, the balloon catheter is of the type described above wherein the longitudinal passage for accommodating the balloon guidewire is central and surrounded by a second co-axial passage for transmitting inflation fluid to a balloon 16, described below.

The balloon catheter carries, near its distal end, a balloon 16. The balloon 16 surrounds the balloon catheter. When the balloon catheter is inflated by the injection of inflation fluid, the balloon expands in all directions from the balloon catheter to impose outward pressure on the inner walls of a blood vessel in which the balloon happens to be located.

Figure 1A is a detail view showing a portion of the apparatus of Figure 1 which is indicated generally by the inset also identified by a reference character 1A. Figure 1A is intended to illustrate the detailed construction of the balloon catheter 14. In Figure 1A, the balloon catheter 14 is a double walled tube. The inner wall 13 of the tube defines a longitudinal passageway 15, which is a central core for accommodating therein the balloon guidewire 12. An outer wall 17 of the balloon catheter 14 is substantially co-axial with the inner wall 13. The space between the outer wall 15 and the inner wall 13 defines a second longitudinal passage extending through the balloon catheter, which passage is indicated by reference characters 17, 17'. The passage 17, 17' communicates with the interior of the balloon 16, and serves as a conduit for transmitting inflation fluid to the interior of the balloon 15. The inflation fluid is supplied by known means.

The second co-axial passage 17 of the balloon catheter communicates with a side port 17a, through which inflation fluid can be injected to inflate the balloon 16. Inflation fluid is injected into the side port 17 by known means such as a syringe.

Preferably, the inflation fluid is a known type of radiopaque liquid. The radiopaque nature of the liquid permits the configuration of the balloon 16 to be imaged by known apparatus and viewed by the physician on a continuous real time basis.

The balloon catheter is made of an extrusion of known relatively flexible plastic, such as nylon. The same is true of the guiding catheter.

It is to be understood that, as an alternative to the balloon catheter construction shown in Figure 1A, the balloon catheter may be of another known configuration in which the longitudinal passages extending through the balloon catheter are not co-axial. In such an alternative embodiment, the longitudinal passages through the balloon catheter are side by side and substantially parallel.

It is to be understood that, in the Figure 1 illustration, the balloon 16 has not yet been extended from the distal end 10a of the guiding catheter 10. Rather, the balloon 16 is, in the Figure 1 illustration, still located within the guiding catheter. The physician, however, by pushing on the proximal end 14a of the balloon catheter can advance the balloon catheter and its associated balloon 16 all the way through the guiding catheter and out the distal end 10a of the guiding catheter, from which the balloon catheter and balloon will track the balloon guidewire until the balloon reaches a location of a constriction which is desired to be re-opened.

When it is desired to inflate the balloon 16, inflation fluid is forced under pressure into the outer longitudinal passage 17, 17' of the balloon catheter by known means, such as a syringe.

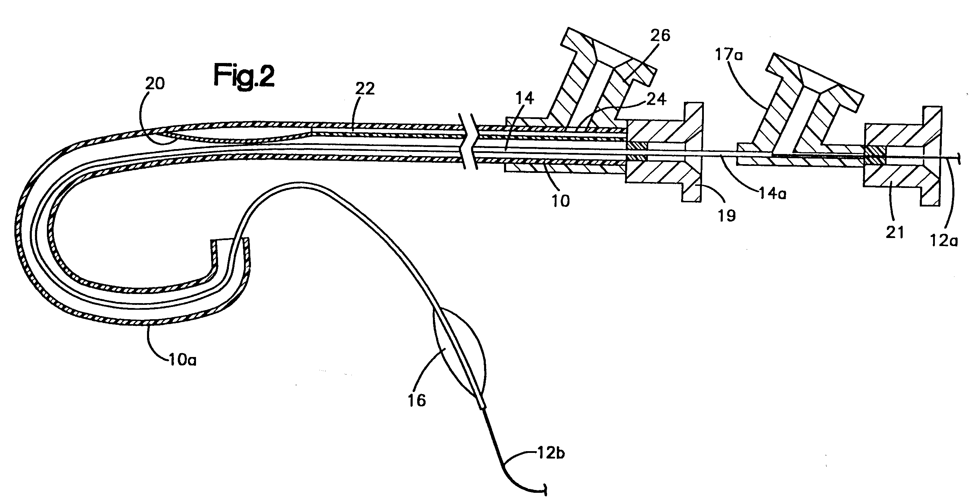

Figure 2 illustrates the apparatus of Figure 1 in which the physician has advanced the balloon catheter to the point at which the balloon 16 attached to the end of the balloon catheter, has exited the distal end of the guiding catheter. In the Figure 2 illustration, it is to be understood that the balloon 16 is located within an artery of the patient, which artery is not shown.

It is also to be understood that the balloon guidewire 12 is pre-located within the patient's artery and extends past the area of blockage or constriction.

As pointed out above, it is also to be understood that the balloon catheter and the balloon guidewire can be moved independently of one another, and independently of the guiding catheter.

Figure 1 also illustrates a holding balloon 20, located near the distal end of the guiding catheter 10. The holding balloon is fixedly mounted to an interior wall of the guiding catheter 10. A conduit 22 communicates with the interior of the holding balloon 20, and extends toward the proximal end of the guiding catheter, terminating in a holding balloon inflation port 26. A syringe, coupled to the holding balloon inflation port, facilitates the injection of balloon inflation fluid into the conduit 22, when it is desired to inflate the holding balloon 20.

The conduit 22 is preferably integrally formed in the wall of the guiding catheter 10 when the guiding catheter is initially manufactured by extrusion. Alternately, the conduit 22 can be an independent piece of tubing, the design of which is well within ordinary skill.

The inclusion of the holding balloon and associated conduit and inflation port in the guiding catheter facilitates the exchange of balloon catheters without attaching an extension to the proximal end of the balloon guidewire, and without requirement for a second individual to assist the physician in effecting the exchange.

In operation, the holding balloon is initially uninflated, and does not substantially obstruct the guiding catheter. In this condition, the physician can advance the balloon catheter past the holding balloon and outwardly from the distal end of the guiding catheter, to a region of arterial constriction which the physician desires to re-open.

If, during this procedure, it becomes apparent that a different sized balloon is going to be required, the holding balloon comes into play. Once a decision is made to exchange the balloon, i.e., the balloon and its balloon catheter, the physician retracts the balloon catheter and its associated balloon to a location at which the balloon is within the guiding catheter, near its distal end, but is located proximally relative to the holding balloon, i.e., to the right of the holding balloon, as shown in Figure 1.

At this point, the physician ceases to retract the balloon catheter and the holding balloon is inflated. The holding balloon, when inflated, fills substantially the entire cross section of the guiding catheter in the region in which the holding balloon is located. This action causes the holding balloon to press the balloon guidewire against the interior wall of the guiding catheter, diametrically opposite the location at which the holding balloon is secured to the interior wall of the guiding catheter. When so inflated, the balloon guidewire is held firmly by friction against movement parallel to the guiding catheter.

Under this condition, the physician can withdraw the balloon catheter completely from the guiding catheter simply by pulling on the proximal end of the balloon catheter. There need be no concern that, during the operation of removing the balloon catheter, the balloon catheter covers the entire proximal end of the balloon guidewire, because there is no longer any need to hold onto the balloon guidewire while the balloon catheter is being withdrawn. The balloon guidewire is, instead of being manually held, held fast by the impingement of the holding balloon within the guiding catheter. Thus, there is no need for a second individual to manually hold the balloon guidewire. Likewise, there is no need to employ a removable extension on the proximal end of the balloon guidewire to enable someone to hold it still by manual means.

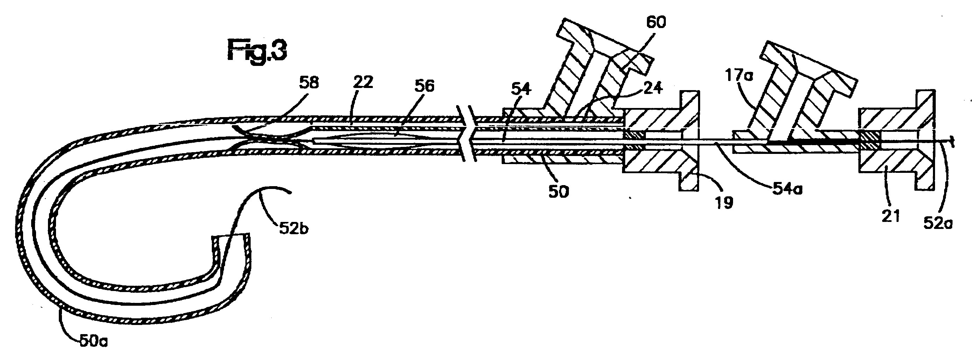

Figure 3 illustrates an alternate embodiment of the invention. Figure 3 shows a guiding catheter 50 having a distal end 50a, a balloon guidewire 52, and a balloon catheter 54, which defines a proximal end 54a and carries a balloon 56 suitable for reopening occluded blood vessels. The balloon catheter, balloon guidewire and balloon 56 are similar to the corresponding elements described in connection with the description of the foregoing embodiments.

The guiding catheter 50, however, is somewhat different from the guiding catheter described above, The guiding catheter 50 includes a holding balloon 58, but the holding balloon has a different configuration and location than the holding balloon described in connection with the foregoing embodiments.

More specifically, and as shown in Figure 3, the holding balloon 58 has an annular or toroidal configuration. The holding balloon 58 defines a central aperture through which can be threaded the balloon guidewire. The outermost surface of the holding balloon 58 is anchored, adhesively or by some other suitable process, about the circumference of the inner wall of the guiding catheter.

When the annular holding balloon is not inflated, the central opening it defines is ample to permit threading therethrough of the balloon guidewire, and subsequently of the balloon catheter.

When it is desired to exchange balloons, and hence the balloon catheter, the balloon catheter is partially withdrawn such that the balloon 56 is located proximal with respect to the holding balloon 58. Then, with only the balloon guidewire extending through the central aperture defined by the annular holding balloon, the annular holding balloon 58 is inflated with inflation fluid delivered by way of an inflation port 60. Inflation of the annular holding balloon 58 causes the interior surfaces of the holding balloon 58 to simultaneously constrict upon the outer circumference of the balloon guidewire. This action causes the annular holding balloon to firmly grip the balloon guidewire about its entire circumference.

An advantage of this annular configuration of the holding balloon 58 over the holding balloon described above, is that the holding balloon impinges about the entire circumference of the balloon guidewire, rather than impinging in merely a portion of the balloon guidewire circumference. Additionally, it is the holding balloon itself which grips the balloon guidewire, rather than the balloon guidewire merely being pressed by the holding balloon against an interior wall surface of the guiding catheter. If the balloon guidewire is merely pressed against one side of the inner wall of the guiding catheter, some slippage of the balloon guidewire can result, particularly if the guiding catheter is made of a material, such as teflon, which has a relatively low coefficient of friction. It is believed that, in some situations, the annular holding balloon 58 effects a greater frictional engagement of the balloon guidewire against longitudinal movement of the balloon guidewire within the guiding catheter.

Preferably, the annular holding balloon 58 is made of constituting a balloon and a relatively high coefficient of friction. The higher coefficient of friction facilitates the holding balloon griping the balloon guidewire.

While the present invention is described above in considerable particularity, it is to be understand that those of ordinary skill in the art may be able to make certain additions or modifications to, or deletions from, the specific embodiments described herein, without departing from the spirit or the scope of the invention, as set forth in the appended claims.

## Claims

1. Catheterization apparatus comprising:
a) a flexible tubular guiding catheter sized for insertion into the vascular system of a patient;
b) a balloon guidewire adapted for longitudinal positioning within said guiding catheter while extending simultaneously from the ends of said guiding catheter;
c) a balloon catheter including a first balloon coupled near the distal end of the balloon catheter, said balloon catheter defining a hollow central longitudinal passage through which said balloon guidewire can be threaded, said balloon catheter also defining a conduit for facilitating transmission of inflation fluid to said first balloon;
d) a generally annular holding balloon defining a central aperture and being disposed within said guiding catheter at a position such that, when said balloon guidewire extends through said central aperture and when said annular holding balloon is inflated, the holding balloon circumferentially constricts about the balloon guidewire, inhibiting longitudinal movement of said balloon guidewire within said guiding catheter; and,
e) means for injecting inflation fluid for inflating said first balloon and said holding balloon.

2. The apparatus of claim 1, wherein the outer circumferential surface of the annular holding balloon is affixed around substantially the entire circumference of a segment of the interior wall of said guiding catheter.

3. The apparatus of claim 1, wherein said means for inflating said holding balloon includes structure defining a conduit communicating with the interior of said holding balloon and extending to a location external to said guiding catheter.

4. The apparatus of claim 3, wherein at least a portion of said conduit is integrally formed in a wall of said guiding catheter.

5. The apparatus of claim 1, wherein said guiding catheter defines a holding balloon inflation port through the wall of said guiding catheter, said port communicating with said conduit.

6. A guiding catheter for use in catheterization, said guiding catheter comprising:
a) a flexible tubular body portion defining internal and external wall surfaces and a hollow central core;
b) a balloon disposed inside said body portion near one end thereof, said balloon being generally annular in configuration and defining a central aperture, said balloon being sized to substantially block said core of said body portion when said balloon is inflated, and
c) structure defining a conduit communicating with the interior of said balloon for introducing inflation fluid into said balloon.

7. A method of inhibiting longitudinal guidewire motion with respect to a guiding catheter in which the guidewire is located, said method comprising the step of:
remotely applying an inwardly pinching force about the circumference of a portion of the guidewire.

8. The method of claim 7, wherein said step of remotely applying an inwardly pinching force comprises inflating a balloon having a generally annular configuration and being located within the guiding catheter, while said guidewire extends through a central opening defined by said balloon, by injecting an inflating fluid into said balloon.

9. Catheterization apparatus comprising:
a) a guiding catheter;
b) a balloon guidewire adapted for insertion into said guiding catheter;
c) a balloon catheter adapted for sliding engagement on the balloon guidewire;
d) a generally annular shaped holding balloon disposed within said guiding catheter, said holding balloon defining a central aperture through which said balloon guidewire can be threaded, and
e) apparatus for introducing inflation fluid into said annular holding balloon to inhibit longitudinal motion, with respect to the guiding catheter, of a balloon guidewire threaded through the central aperture defined by said annular holding balloon.

10. An apparatus for inhibiting longitudinal movement of a guidewire in a guiding catheter of a cardiac catheterization apparatus, said apparatus comprising:
apparatus for remotely applying a constricting pinching force about the circumference of the guidewire.
